# EUROPEAN PATENT SPECIFICATION

(11) **EP 3 449 950 B1**
(45) Date of publication and mention of the grant of the patent: **10.03.2021**
(21) Application number: 17843740.6
(22) Date of filing: 25.08.2017
(51) Int. Cl.: A61L 9/015, A47L 23/20, A61L 2/20, A61L 2/14, A61L 9/22, C01B 13/11, H05H 1/24, A43B 7/00, A43B 17/00, A43B 19/00

(54) **SHOE CLEANER**
SCHUHREINIGER
DISPOSITIF DE NETTOYAGE DE CHAUSSURE

(30) Priority: 26.08.2016 JP 2016166333
(43) Date of publication of application: 06.03.2019
(73) Proprietor: CREATIVE TECHNOLOGY CORPORATION, Kawasaki-shi, Kanagawa 213-0034 (JP)
(72) Inventor: KOYAMA Satomi, Kawasaki-shi Kanagawa 213-0034 (JP); HIRANO Shinsuke, Kawasaki-shi Kanagawa 213-0034 (JP); TATSUMI Yoshiaki, Kawasaki-shi Kanagawa 213-0034 (JP); LUO Li, Kawasaki-shi Kanagawa 213-0034 (JP); TSUBOI Kazuki, Kawasaki-shi Kanagawa 213-0034 (JP); KUSANO Mutsumi, Kawasaki-shi Kanagawa 213-0034 (JP)
(74) Representative: Winter, Brandl, Fürniss, Hübner, Röss, Kaiser, Polte - Partnerschaft mbB
(86) International application number: PCT/JP2017/030590
(87) International publication number: WO 2018/038264

(56) References cited:
- EP-A1- 2 953 431
- EP-A1- 3 299 034
- WO-A1-2009/003613
- JP-A- 2001 087 367
- JP-A- 2006 228 513
- KR-A- 20110 043 030
- KR-A- 20150 022 286
- US-A1- 2016 242 620

## Description

### Technical Field

The present invention relates to a shoe cleaner capable of sterilizing and deodorizing a shoe by ozone.

### Background Art

Conventionally, as techniques of this kind, for example, there are techniques described in some patent literatures, which are referred to below.

The technique described in JP 2001 314250 A is a device with an ozone generator installed inside a shoe locker. According to this device, ozone generated by the ozone generator is circulated throughout the shoe locker to sterilize and deodorize shoes, etc.

The technique described in JPH 11 104223 A is a device including an ozone generator and a power supply control device incorporated in an outer case. According to this device, the outer case is inserted into a shoe, and by generating ozone into a wide space of the outer case from the ozone generator, ozone is emitted into the shoe through holes of the outer case, and sterilization, etc., are applied to the inside of the shoe by the emitted ozone.

On the other hand, the technique described in JP 2006 034925 A is a device including not only an ozone generator but also a fan, etc. According to this device, not only is ozone generated into a wide space of the outer case from the ozone generator, but the generated ozone is also forcibly blown out by using the fan or a nozzle so as to efficiently perform sterilization, etc.

KR 2011 0043030 A discloses a shoe insole including an insole main body, a piezoelectric element accommodated in the insole main body and a plasma generator. When a user walks wearing shoes equipped with such shoe insoles, each piezoelectric element receives a pressure and generates a voltage, which is supplied for the plasma generator. The plasma generators, when supplied with the voltage, generate plasma, which contributes to sterilization of the shoes.

WO 2009/003613 A1 discloses a flexible dielectric material to create cold plasma. As one example of applications of this material, a shoe insert is listed.

EP 2 953 431 A1 also discloses a plasma generator that employs dielectric layer made of flexible polymer resin layers.

KR 2015 0022286 A discloses a shoes sterilizer including a housing, a plasma module and a blower. In a bottom surface of the casing , protrusions are formed. Plasma that has generated at the plasma module and that has been pumped by the blower circulates through space between the bottom surface and a shoe sole.

EP 3 299 034 A1 discloses a sterilization case for sterilizing a mobile communication device by generating and providing ozone JP 2006 228513 A discloses a sheet-like ion generating device for sterilisation.

### Summary of the Invention

### Technical Problem

However, the conventional techniques described above have the following problems.

The technique described in JP 2001 314250 A is a device to sterilize the inside of a shoe locker, which is a comparatively large space, so that in order to sterilize and deodorize shoes, etc., inside the shoe locker by ozone, an amount of ozone to be emitted to the inside of the shoe locker needs to be large. Therefore, a user using this device may be exposed to high concentrations of ozone during use.

On the other hand, in the techniques described in JPH 11 104223 A and JP 2006 034925 A, there is little likelihood that a user is exposed to high concentrations of ozone during use, however, the device includes an ozone generator, etc., incorporated inside an outer case, so that the device itself becomes large in size.

In particular, in the technique described in JP 2006 034925 A, not only an ozone generator but also units such as a fan, a nozzle, etc., need to be provided inside the outer case, so that the device itself may further increase in size. Further, not only electric power to activate the ozone generator but also electric power to activate the fan are necessary, so that power consumption of the device during operation increases.

The present invention has been made to solve the problems described above, and an object thereof is to provide a shoe cleaner which can be used by a user without exposing the user to high concentrations of ozone, and moreover which is compact, is easy to use, and has low power consumption.

### Solution to the Problems

In order to solve the above-described problems, the invention of claim 1 is directed to a shoe cleaner including an ozone generating body capable of being inserted into a shoe from a wearing opening of the shoe, and a case body attached to a front surface of the ozone generating body and capable of being inserted into the shoe integrally with the ozone generating body, wherein the ozone generating body is formed of a sheet-like substrate having the front surface attached to a bottom portion of the case body, and an electrode sheet having a front surface stuck to a back surface of the substrate, and including a pair of electrodes, at least one of the pair of electrodes being covered by a dielectric and at least the dielectric being formed of a polymeric resin, and a voltage supply portion capable of supplying a voltage necessary to generate ozone to the pair of electrodes of the electrode sheet is housed inside the case body.

With this configuration, a user can insert the entire shoe cleaner to the inside of a shoe from a wearing opening of the shoe in a state where the back surface of the ozone generating body is turned toward a shoe sole side.

In this state, when the voltage supply portion housed in the case body is activated, a desired voltage is supplied to the electrode sheet of the ozone generating body, and ozone is emitted from the electrode sheet.

At this time, the electrode sheet of the ozone generating body faces the shoe sole, so that ozone emitted from the electrode sheet is directly blown to the shoe sole, and the shoe sole is sufficiently sterilized and deodorized.

That is, with the shoe cleaner of the present invention, ozone is only generated into a narrow space, that is, inside a shoe, so that without generating a large amount of high concentrations of ozone, the inside of the shoe can be efficiently sterilized and deodorized by a small amount of ozone. As a result, the shoe cleaner can be used without exposing a user to high concentrations of ozone. Further, because ozone to be generated can be small in amount, a voltage to be supplied from the voltage supply portion to the electrode sheet can be accordingly lowered, and as a result, power consumption can be reduced.

The shoe cleaner of the present invention is configured to include an ozone generating body capable of being inserted into a shoe and a case body attached to the ozone generating body and capable of being inserted into the shoe integrally with the ozone generating body, so that the shoe cleaner as a whole can be downsized. In addition, there is no need to provide a space to generate ozone and a space to accommodate a blowing fan into the case body as in the devices described in JPH 11 104223 A and JP 2006 034925 A described above, so that further downsizing and simplification of the configuration can be accordingly realized.

The downsized shoe cleaner enables space saving of a storage area for the shoe cleaner when not used.

In the shoe cleaner of the present invention, at least the dielectric is formed of a polymeric resin in the electrode sheet, so that there is no fear of cracking that occurs in the case of an ozone generating body formed of ceramic or glass, and the shoe cleaner can be easily handled by a general user. That is, by taking advantage of the properties of the polymeric resin, the ozone generating body can be reduced in thickness and weight, and the electrode sheet can be easily processed into an arbitrary shape.

The invention of claim 2 is directed to the shoe cleaner according to claim 1, in which at least one of the electrode sheet and the substrate is formed of a flexible material.

With this configuration, by forming both of the electrode sheet and the substrate of flexible materials, when the ozone generating body is inserted into a shoe, the ozone generating body deforms to follow the shape of the inside of the shoe. Therefore, the shoe cleaner can be smoothly arranged at a desired position inside the shoe.

The invention of claim 3 is directed to the shoe cleaner according to claim 1 or 2, in which the dielectric of the electrode sheet is formed of a polymeric resin with a relative permittivity of 3 or more and a dielectric breakdown voltage of 15 kV/mm or more.

With this configuration, the electrode sheet becomes a strong member that can withstand a high voltage, and moreover, plasma to generate ozone is easily generated.

The invention of claim 4 is directed to the shoe cleaner according to any of claims 1 to 3, in which the substrate is formed of a material with a Young's modulus of 40 MPa or less.

The invention of claim 5 is directed to the shoe cleaner according to any of claims 1 to 4, in which a spacer to keep a distance of 10 mm or less between a back surface of the ozone generating body and a shoe sole is provided on the back surface of the ozone generating body.

With this configuration, in particular, a portion that has a rough surface and is difficult to sterilize and deodorize, such as a shoe inside and an insole, can be reliably sterilized and deodorized.

The invention of claim 6 is directed to the shoe cleaner according to claim 5, in which the spacer is a projection with a height of 10 mm or less provided to project from the back surface of the electrode sheet.

The invention of claim 7 is directed to the shoe cleaner according to claim 5, in which the spacer is a net-like body with a thickness of 10 mm or less attached to the back surface of the electrode sheet.

The invention of claim 8 is directed to the shoe cleaner according to any of claims 1 to 4, in which a sectional shape of the ozone generating body is corrugated.

With this configuration, gaps between the corrugated ozone generating body and a shoe sole are filled with a large amount of air, and the ozone generating efficiency of the electrode sheet is improved. As a result, the sterilization effect increases. Further, by forming the ozone generating body into not a planar shape but a corrugated shape and installing the ozone generating body along a shoe sole, a large back surface area can be provided for the ozone generating body.

The invention of claim 9 is directed to the shoe cleaner according to claim 8, in which a height of corrugation of the ozone generating body is set to 10 mm or less.

With this configuration, in particular, a portion that has a rough surface and is difficult to sterilize and deodorize, such as a shoe inside and an insole, can be reliably sterilized and deodorized.

The invention of claim 10 is directed to the shoe cleaner according to any of claims 1 to 9, in which the ozone generating body is provided with a plurality of holes penetrating through the substrate and the electrode sheet from the front surface side to the back surface side.

With this configuration, a large amount of air enters into the back surface side from the front surface side of the ozone generating body through the plurality of holes. As a result, the ozone generating efficiency is improved, and the sterilization effect and the deodorization effect can be increased.

The invention of claim 11 is directed to the shoe cleaner according to any of claims 1 to 10, in which the pair of electrodes in the electrode sheet are respectively formed into comb shapes, and combs of the pair of electrodes engage with each other while keeping a fixed interval between them.

The invention of claim 12 is directed to the shoe cleaner according to any of claims 1 to 10, in which one of the pair of electrodes in the electrode sheet is housed inside the dielectric, and either the other electrode having a large number of holes or the other electrode having a comb shape is located on the dielectric so as to face the one electrode.

### Effects of the Invention

As described in detail hereinbefore, according to the present invention, an excellent effect can be obtained in which ozone is only generated into a narrow space, that is, inside a shoe, so that various portions inside the shoe can be sterilized and deodorized by a small amount of ozone without generating a large amount of high concentrations of ozone. In addition, there is also an effect in which the effect of sterilization and deodorization can be obtained by generating a small amount of ozone, so that a voltage to be supplied from the voltage supply portion to the electrode sheet can be accordingly lowered, and as a result, an effect of reducing power consumption can also be obtained.

In the present invention, downsizing of the external shape and simplification of the configuration can be realized, so that space saving of a storage area and a further reduction in power consumption can be realized.

Further, in the present invention, in the electrode sheet, at least the dielectric is formed of a polymeric resin, so that there is no fear of cracking and the shoe cleaner can be easily handled by a general user. Because of the properties of the polymeric resin, the ozone generating body can be reduced in thickness and weight, and the electrode sheet can be easily processed into an arbitrary shape.

According to the invention of claim 2, by forming both of the electrode sheet and the substrate of flexible materials, the shoe cleaner can be smoothly arranged at a desired position inside a shoe.

According to the invention of claim 3, the life of the electrode sheet can be extended, and ozone generation can be facilitated.

In particular, according to the inventions of claims 5 to 7, in particular, a portion that has a rough surface and is difficult to sterilize and deodorize, such as a shoe inside and an insole, can be reliably sterilized and deodorized.

According to the inventions of claims 8 to 10, the ozone generating efficiency of the electrode sheet can be improved.

In particular, according to the invention of claim 8, a large back surface area can be secured for the ozone generating body.

According to the invention of claim 9, in particular, a portion that has a rough surface and is difficult to sterilize and deodorize, such as a shoe inside and an insole, can be reliably sterilized and deodorized.

### Brief Description of the Drawings

Fig. 1 is a perspective view showing a shoe cleaner according to a first embodiment of the present invention.
Fig. 2 is a plan view of the shoe cleaner, viewed from a front surface side.
Fig. 3 is a plan view of the shoe cleaner, viewed from a back surface side.
Fig. 4 is a sectional view taken along arrow A-A in Fig. 2.
Fig. 5 is a partially cut-away schematic plan view of an ozone generating body.
Fig. 6 is a schematic sectional view to describe an ozone generating function by a boost circuit.
Fig. 7 is a schematic sectional view showing a state when the shoe cleaner is inserted into a shoe.
Fig. 8 is a schematic sectional view to describe sterilization and deodorization effects of the shoe cleaner.
Fig. 9 is a schematic sectional view showing a state when the shoe cleaner is taken out from the inside of the shoe.
Fig. 10 is a side view showing a shoe cleaner according to a first modification of the first embodiment.
Figs. 11 are side views showing a shoe cleaner according to a second modification of the first embodiment, in which Fig. 11(a) shows a state of an ozone generating body during use of the shoe cleaner, and Fig. 11(b) shows a state where the ozone generating body is folded.
Fig. 12 is a view showing a standing state of a shoe cleaner according to a third modification of the first embodiment.
Fig. 13 is an electrical connection state diagram showing a shoe cleaner according to a fourth modification of the first embodiment.
Fig. 14 is an electric block diagram showing an essential portion of a shoe cleaner according to a fifth modification of the first embodiment.
Fig. 15 is a plan view showing a shoe cleaner according to a second embodiment of the present invention.
Fig. 16 is a schematic plan view showing an electrical connection state of the second embodiment.
Fig. 17 is a schematic sectional view showing a usage example of the shoe cleaner.
Fig. 18 is a side view of a shoe cleaner according to a third embodiment of the present invention.
Fig. 19 is a plan view of the shoe cleaner, viewed from a back surface side.
Fig. 20 is a schematic sectional view showing a state where the shoe cleaner is inserted into a shoe.
Figs. 21 are transferred images of photographs showing survival states of bacteria.
Fig. 22 is a diagram showing a relationship between a survival rate of bacteria and a distance.
Fig. 23 is a plan view of a shoe cleaner according to a fourth embodiment of the present invention, viewed from the back surface.
Fig. 24 is a side view showing a shoe cleaner according to a fifth embodiment of the present invention.
Fig. 25 is a plan view of a shoe cleaner according to a sixth embodiment of the present invention.
Fig. 26 is a side view showing the part of an ozone generating body in section.
Fig. 27 is a schematic sectional view showing an essential portion of a shoe cleaner according to a seventh embodiment of the present invention.
Fig. 28 is an exploded perspective view of the essential portion shown in Fig. 27.
Figs. 29 are plan views showing modifications of an electrode applied in the seventh embodiment, in which Fig. 29(a) shows a first modification of the seventh embodiment, and Fig. 29(b) shows a second modification of the seventh embodiment.

### Description of the Embodiments

The best mode of the present invention will hereinafter be described with reference to the accompanying drawings.

### (First Embodiment)

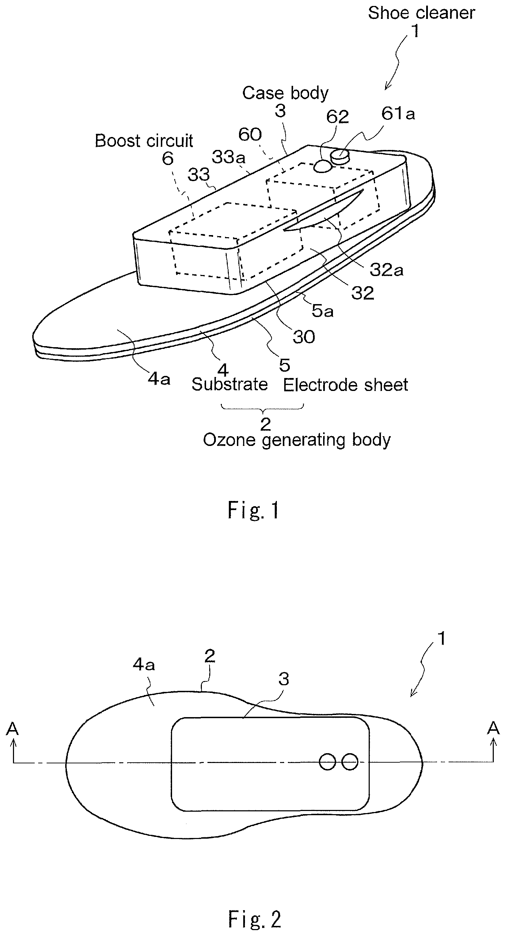

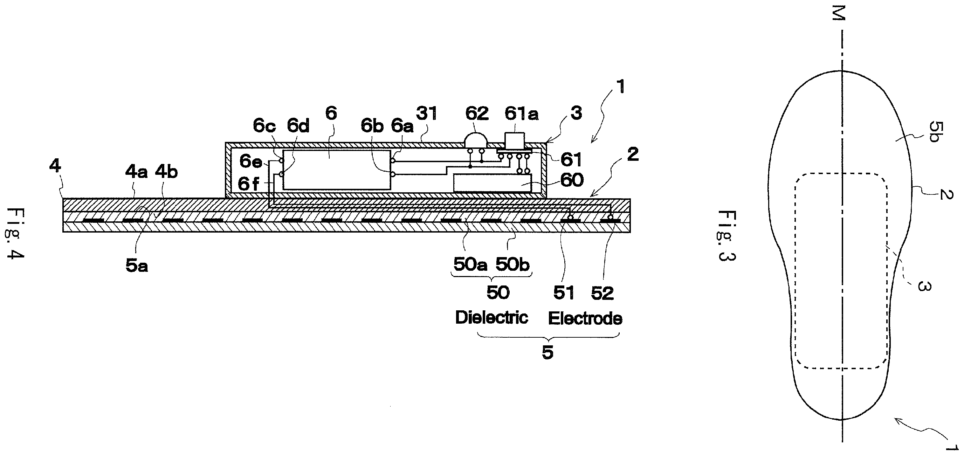

Fig. 1 is a perspective view showing a shoe cleaner according to a first embodiment of the present invention, Fig. 2 is a plan view of the shoe cleaner, viewed from a front surface side, and Fig. 3 is a plan view of the shoe cleaner, viewed from a back surface side.

As shown in Fig. 1, the shoe cleaner 1 of the present embodiment is a device to sterilize and deodorize the inside of a shoe, and includes an ozone generating body 2 and a case body 3.

The ozone generating body 2 is a sheet-like member to generate ozone, and has a shape capable of being inserted into a shoe from a wearing opening of the shoe.

In detail, as shown in Fig. 2, the shape of the ozone generating body 2 is set to an overall shape of an insole of the shoe. Further, as shown in Fig. 3, the shape of the ozone generating body 2 is formed to be symmetrical with respect to a central axis M. Accordingly, the ozone generating body 2 can be inserted into either of left and right shoes, and moreover, when inserted, the ozone generating body 2 reaches the vicinity of a tip end or a side end of a toe of the shoe.

The ozone generating body 2 having this overall shape is configured by sticking together a sheet-like substrate 4 and an electrode sheet 5 that have shapes equal to each other.

The substrate 4 is a flexible member whose front surface 4a is stuck to a bottom wall 30 serving as a bottom portion of the case body 3.

This substrate 4 is formed of a material with a Young's modulus of 40 MPa (megapascal) or less, and has an elastic property corresponding to hardness Hs (Shore hardness) of 70 of rubber.

In detail, rubber such as silicone rubber or ethylene propylene rubber or a sponge-like material such as polyurethane foam or polyethylene foam, having a Young's modulus of 40 MPa or less, is used as the substrate 4.

The electrode sheet 5 is a flexible sheet member that generates ozone, and has a front surface 5a stuck to a back surface of the substrate 4, and the entire electrode sheet 5 is supported by the substrate 4.

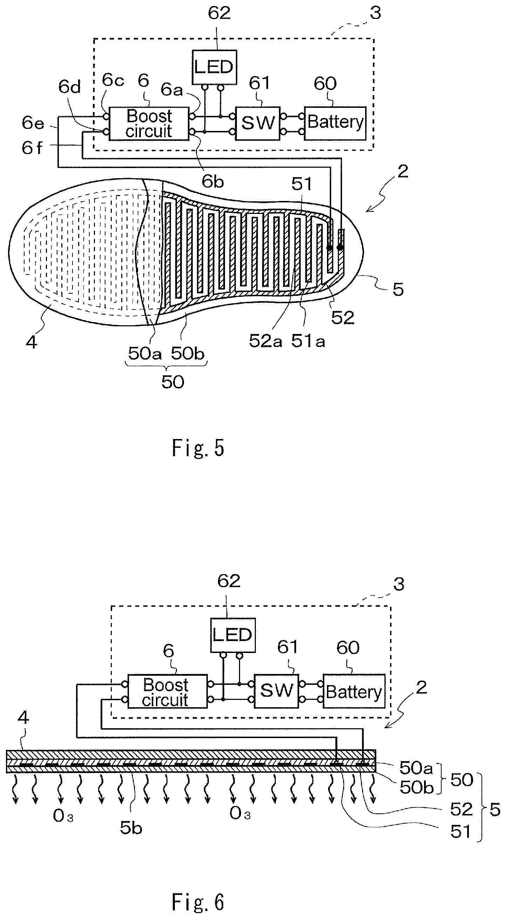

Fig. 4 is a sectional view taken along arrow A-A in Fig. 2, and Fig. 5 is a partially cut-away schematic plan view of the ozone generating body 2.

As shown in Fig. 4, the electrode sheet 5 is formed of a sheet-like dielectric 50, and a pair of electrodes 51 and 52 covered by the dielectric 50.

In detail, the dielectric 50 consists of two layers including dielectric layers 50a and 50b, and the electrodes 51 and 52 are formed on the dielectric layer 50b as a lower layer, and the dielectric layer 50a as an upper layer is laminated on the dielectric layer 50b so as to cover the electrodes 51 and 52.

This electrode sheet 5 is entirely formed of a polymeric resin.

In detail, the dielectric layers 50a and 50b are formed of a polymeric resin with a permittivity of 3 or more and a dielectric breakdown voltage of 15 kV/mm (kilovolts/millimeters) or more. In the present embodiment, as such a polymeric resin, any of polyimide, silicone, PET (polyethylene terephthalate), and vinyl chloride is used. The electrodes 51 and 52 are also formed of a conductive polymer being a polymeric resin.

Moreover, as shown in Fig. 5, each of the pair of electrodes 51 and 52 is formed into a comb shape. That is, the plurality of combs 51a and 52a engage with each other while keeping a fixed interval between them.

On the other hand, as shown in Fig. 1, the case body 3 is stuck to the front surface of the ozone generating body 2, that is, the front surface 4a of the substrate 4, and recess portions 32a and 33a to allow fingers to pinch the case body 3 are formed on both side walls 32 and 33 of the case body 3.

Accordingly, by pinching the recess portions 32a and 33a, the case body 3 can be inserted together with the ozone generating body 2 into a shoe.

In this case body 3, as shown in Fig. 4, a boost circuit 6 as a voltage supply portion is housed.

This boost circuit 6 is a circuit to supply a voltage necessary for ozone generation to the electrode sheet 5. In the present embodiment, the shoe cleaner 1 is assumed to be used in a location near which no commercial power supply is present, such as a shoe locker and an entrance, etc., so that a battery 60 is used as a power supply.

In detail, as shown in Fig. 4 and Fig. 5, the battery 60 is housed inside the case body 3, and electrically connected to input terminals 6a and 6b of the boost circuit 6 via a switch 61.

In the present embodiment, the battery 60 is a power supply to supply a voltage of, for example, 3V DC (Direct Current) to the boost circuit 6, and the supply can be turned on or off by the switch 61.

The switch 61 is fitted to a ceiling wall 31 of the case body 3, a push button 61a is a portion to operate the switch 61, and a head portion of the push button projects upward from the ceiling wall 31.

The boost circuit 6 is connected to the battery 60 via the switch 61, and output terminals 6c and 6d of the boost circuit 6 are respectively electrically connected to the electrodes 51 and 52 of the electrode sheet 5 through wirings 6e and 6f.

Accordingly, the boost circuit 6 can boost a DC voltage from the battery 60 to a voltage necessary for ozone generation and then supply the voltage to the electrodes 51 and 52 of the electrode sheet 5. In the present embodiment, the boost circuit 6 boosts the DC voltage of 3V input from the battery 60 to, for example, an AC (Alternating Current) voltage or a pulsed voltage of 2 kV to 10 kV, and applies the voltage to the electrodes 51 and 52 of the electrode sheet 5.

Moreover, an LED lamp 62 is provided between the boost circuit 6 and the switch 61. In detail, the LED lamp 62 is fitted to the ceiling wall 31 of the case body 3, and a head portion of the LED lamp projects from the ceiling wall 31.

In Fig. 4, the wirings 6e and 7f connecting the output terminals 6c and 6d of the boost circuit 6 and the electrodes 51 and 52 of the electrode sheet 5 are described inside the substrate 4, however, this description is for easy understanding of the wiring state, and in actuality, the wirings 6e and 6f are formed on the front surface 4a of the substrate 4 and the front surface 5a of the electrode sheet 5.

Fig. 6 is a schematic sectional view to describe an ozone generating function by the boost circuit 6.

Since the boost circuit 6 is connected to the electrode sheet 5 as described above, as shown in Fig. 6, by turning on the switch 61, a DC voltage of 3V of the battery 60 is input to the boost circuit 6, and an AC or pulsed high voltage of 2 kV to 10 kV is supplied from the boost circuit 6 to the electrodes 51 and 52 of the electrode sheet 5. As a result, discharge occurs between the electrodes 51 and 52, and ozone O₃ is emitted downward from a back surface 5b of the electrode sheet 5. In addition, the LED lamp 62 is turned on concurrently with the turning-on operation of the switch 61, and a user can visually recognize that the shoe cleaner 1 is operating based on the turning-on of the LED lamp 62. Then, the LED lamp 62 is turned off by a turning-off operation of the switch 61.

As described above, the shoe cleaner 1 of the present embodiment is simply configured to include the ozone generating body 2 and the case body 3 capable of being inserted into a shoe, so that the shoe cleaner 1 as a whole is small. Therefore, a storage area for the shoe cleaner 1 when not used can be narrowed.

Moreover, since the electrode sheet 5 is formed of a polymeric resin, processing of the ozone generating body 2 is easy.

Next, a usage example of the shoe cleaner of the present embodiment is described.

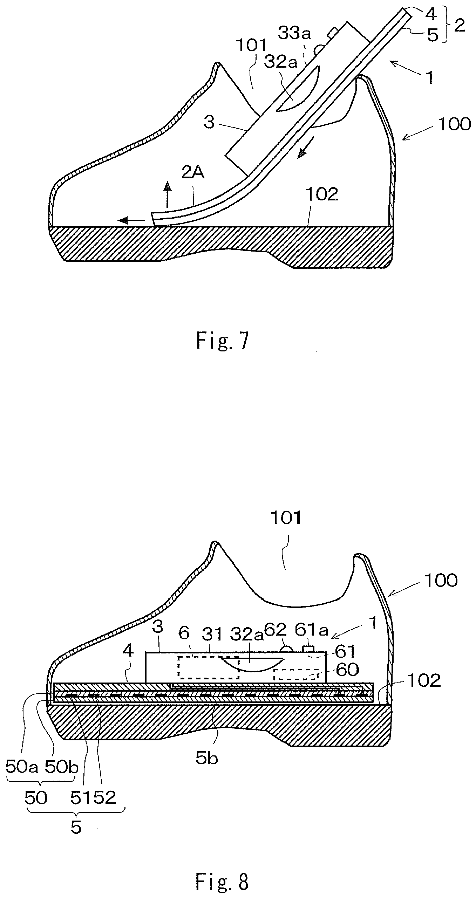

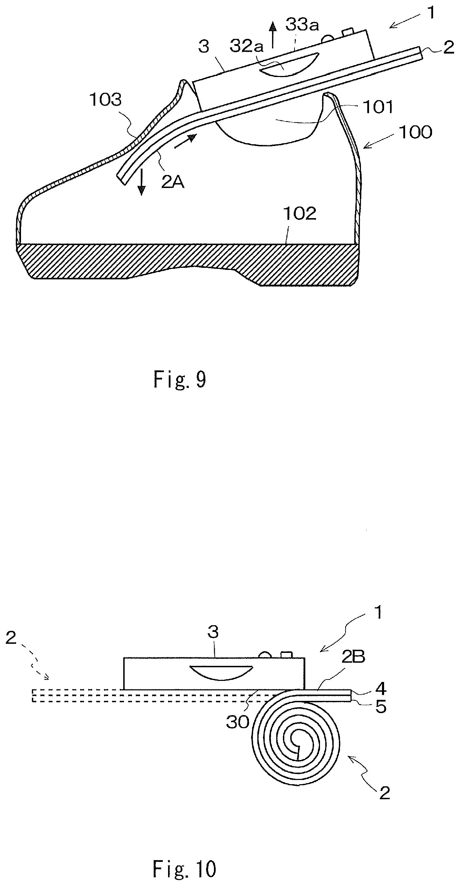

Fig. 7 is a schematic sectional view showing a state when the shoe cleaner is inserted into a shoe, Fig. 8 is a schematic sectional view to describe sterilization and deodorization effects of the shoe cleaner, and Fig. 9 is a schematic sectional view showing a state when the shoe cleaner is taken out from the inside of the shoe.

As shown in Fig. 7, when the shoe cleaner 1 is inserted into a shoe 100, the recess portions 32a and 33a of the case body 3 are pinched by fingers and the entire shoe cleaner 1 is lifted up. Then, with the ozone generating body 2 turned downward, the shoe cleaner 1 is inserted into the shoe 100 from a wearing opening 101.

Then, a toe side portion 2A of the ozone generating body 2 butts against a shoe sole 102. At this time, since the substrate 4 of the ozone generating body 2 is formed of a flexible member such as silicone rubber with a hardness Hs of 50, and the electrode sheet 5 is formed of flexible polymeric resins including polyimide or the like and conductive polymer or the like, so that when the ozone generating body 2 is further pushed down to the shoe sole 102 side, the toe side portion 2A deforms to follow the shape of the shoe sole 102.

Therefore, by pushing the entire shoe cleaner 1 into the shoe 100, the ozone generating body 2 enters the inside of the shoe 100 while deforming. And, finally, the ozone generating body 2 is positioned horizontally on the shoe sole 102 of the shoe 100.

As a result, as shown in Fig. 8, the entire electrode sheet 5 of the ozone generating body 2 faces the shoe sole 102 of the shoe 100.

In this state, the push button 61a projecting from the ceiling wall 31 of the case body 3 is depressed to turn on the switch 61. Then, the LED lamp 62 of the case body 3 is turned on, and an AC or pulsed high voltage of 2 kV to 10 kV is supplied from the boost circuit 6 to the electrodes 51 and 52 of the electrode sheet 5. Accordingly, ozone O₃ (refer to Fig. 6) is emitted from the back surface 5b of the electrode sheet 5 of the ozone generating body 2 toward the shoe sole 102 side.

At this time, since the dielectric layers 50a and 50b of the electrode sheet 5 are formed of the polymeric resin with a permittivity of 3 or more and a dielectric breakdown voltage of 15 kV/mm or more, the electrode sheet 5 can, without being broken, directly blow a sufficient amount of ozone O₃ for sterilization, etc., to the shoe sole 10 facing the electrode sheet 5. As a result, ozone O₃ is sufficiently spread throughout the inside of the shoe 100 and sterilizes and deodorizes various portions inside the shoe 100.

Moreover, ozone O₃ is only generated into a narrow space, that is, inside the shoe 100, so that it is not necessary to generate high concentrations of ozone. Therefore, the inside of the shoe 100 can be efficiently sterilized and deodorized by a small amount of ozone O₃, and the voltage to be supplied from the boost circuit 6 to the electrode sheet 5 can be accordingly lowered.

After the sterilization and deodorization of the inside of the shoe 100 are finished, the push button 61a of the case body 3 is depressed again to turn off the switch 61. Then, the LED lamp 62 is turned off, and emission of ozone from the electrode sheet 5 of the ozone generating body 2 is stopped.

In this state, the recess portions 32a and 33a of the case body 3 are pinched and the shoe cleaner 1 can be taken out from the inside of the shoe 100.

Meanwhile, as shown in Fig. 9, when the shoe cleaner 1 is taken out from the inside of the shoe 100, the toe side portion 2A of the ozone generating body 2 may come into contact with a shoe lining 103 (portion with which the instep comes into contact). However, the substrate 4 and the electrode sheet 5 of the ozone generating body 2 are formed of such flexible members as described above, so that by further pulling out and moving the ozone generating body 2, the toe side portion 2A deforms to follow the shape of the shoe lining 103. As a result, the shoe cleaner 1 can be smoothly taken out from the wearing opening 101 of the shoe 100.

### (First Modification)

Fig. 10 is a side view showing a shoe cleaner according to a first modification of the first embodiment.

As shown in Fig. 10, in this modification, the substrate 4 and the electrode sheet 5 of the ozone generating body 2 are formed of extremely highly flexible members, and part of a heal side portion 2B of the ozone generating body 2 is stuck to the bottom portion 30 of the case body 3.

Accordingly, during use of the shoe cleaner 1, as shown by the dashed lines, the ozone generating body 2 can be spread horizontally and used. When the shoe cleaner 1 is not used, as shown by the solid lines, by rolling up the ozone generating body 2, the shoe cleaner 1 can be stored in a narrow space.

### (Second Modification)

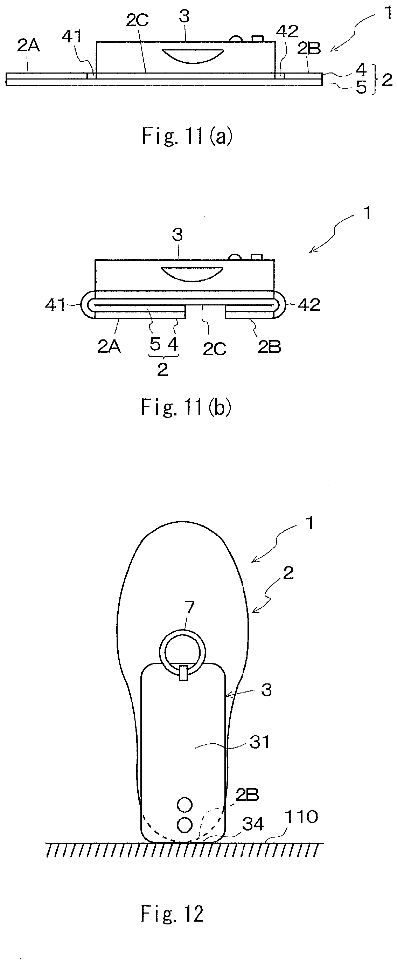

Figs. 11 are side views showing a shoe cleaner according to a second modification of the first embodiment, in which Fig. 11(a) shows a state of the ozone generating body 2 during use of the shoe cleaner, and Fig. 11(b) shows a state where the ozone generating body 2 is folded.

As specifications of the shoe cleaner 1, in some cases, the shoe cleaner is desired to have a configuration in which a flexible electrode sheet 5 is securely supported by a hard substrate 4 that is not flexible and in which storage performance of the shoe cleaner is improved.

The shoe cleaner 1 according to the present modification is configured so as to adapt to such a case. In detail, as shown in Fig. 11(a), the substrate 4 of the ozone generating body 2 is separated into a toe side portion 2A, a heel side portion 2B, and an arch side portion 2C. The portions 2A and 2C of the substrate 4 are supported rotatably by a joint member 41, and the portions 2B and 2C of the substrate 4 are supported rotatably by a joint member 42.

Accordingly, during use of the shoe cleaner 1, the substrate 4 securely supports the electrode sheet 5, and the ozone generating body 2 can be set horizontally. When the shoe cleaner 1 is not used, as shown in Fig. 11(b), by folding the toe side portion 2A and the heel side portion 2B to the side under the arch side portion 2C, the shoe cleaner 1 can be made compact.

As the joint members 41 and 42, elastic rubbers, coil springs, leaf springs, and hinges, etc., can be used.

### (Third Modification)

Fig. 12 is a view showing a standing state of a shoe cleaner according to a third modification of the first embodiment.

In the shoe cleaner 1 of the present modification, as shown in Fig. 12, a ring 7 is attached to the ceiling wall 31 of the case body 3.

Accordingly, by hooking the ring 7 on a hook, etc., provided in a shoe locker, etc., the shoe cleaner 1 can be stored inside the shoe locker, etc.

Moreover, this modification is set so that a rear wall portion 34 of the case body 3 and a rear end of the heel side portion 2B of the ozone generating body 2 substantially match each other.

Accordingly, the shoe cleaner 1 can be placed vertically on a floor 110, etc.

### (Fourth Modification)

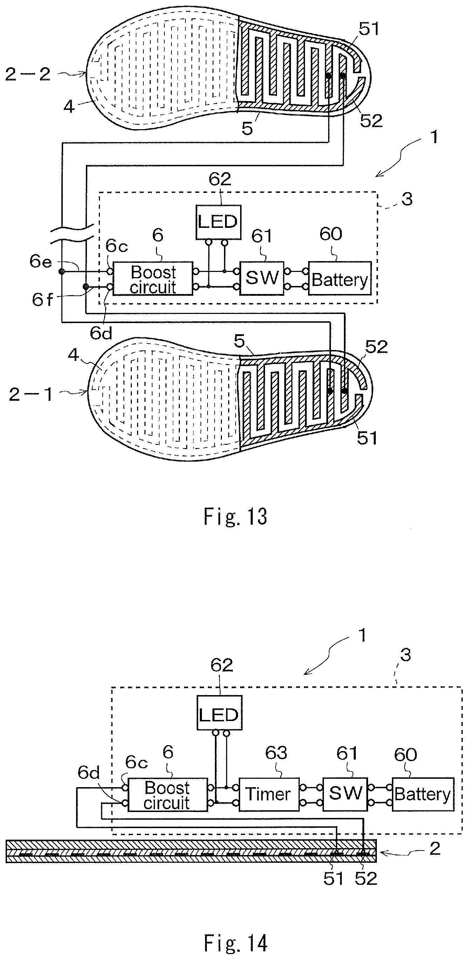

Fig. 13 is an electrical connection state diagram showing a shoe cleaner according to a fourth modification of the first embodiment.

The shoe cleaner of the first embodiment described above is configured to sterilize and deodorize each shoe of a pair of shoes at a time.

As shown in Fig. 13, the shoe cleaner 1 of the present modification includes an ozone generating body 2-1 for a left shoe and an ozone generating body 2-2 for a right shoe. The wirings 6e and 6f from the output terminals 6c and 6d of the boost circuit 6 inside the case body 3 are connected to both of the electrodes 51 and 52 of the ozone generating body 2-1 and the electrodes 51 and 52 of the ozone generating body 2-2.

Accordingly, by inserting the ozone generating body 2-1 into a shoe for a left foot and inserting the ozone generating body 2-2 into a shoe for a right foot, and turning on the switch 61, the left and right shoes can be simultaneously sterilized and deodorized.

### (Fifth Modification)

Fig. 14 is an electric block diagram showing an essential portion of a shoe cleaner according to a fifth modification of the first embodiment.

In the shoe cleaner 1 of the present modification, a timer is provided inside the case body 3.

In detail, as shown in Fig. 14, the timer 63 is interposed between the switch 61 and the boost circuit 6.

Accordingly, when the switch 61 is turned on, the timer 63 and the boost circuit 6 are activated. Then, at the same time as the elapse of a drive time (for example, 2 hours) set in the timer 63, the timer 63 makes the boost circuit 6 automatically stop.

Moreover, in the first embodiment described above and the present modification, the LED lamp 62 may be provided with a function of detecting a remaining battery level of the battery 60 and notifying a time to replace the battery 60 by blinking when the remaining battery level becomes low.

### (Second Embodiment)

Next, a second embodiment of the present invention is described.

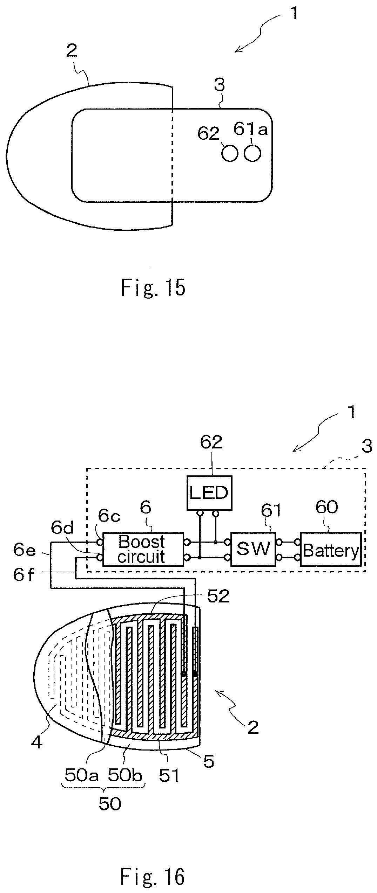

Fig. 15 is a plan view showing a shoe cleaner according to a second embodiment of the present invention, and Fig. 16 is a schematic plan view showing an electrical connection state of the present embodiment.

In the shoe cleaner according to the first embodiment described above, as shown in Fig. 2, the shape of the ozone generating body 2 is set to an overall shape of an insole of a shoe, however, the shoe cleaner 1 according to the present embodiment is different from the first embodiment described above in that, as shown in Fig. 15, the shape of the ozone generating body 2 is set to a shape of a toe side portion of an insole of a shoe.

In detail, as shown in Fig. 16, the substrate 4 and the electrode sheet 5 of the ozone generating body 2 are respectively formed into the same shape, and are respectively formed into a shape of a toe side portion of an insole of a shoe. The electrodes 51 and 52 are formed between the dielectric layers 50a and 50b, and are connected to the output terminals 6c and 6d of the boost circuit 6 inside the case body 3 through the wirings 6e and 6f.

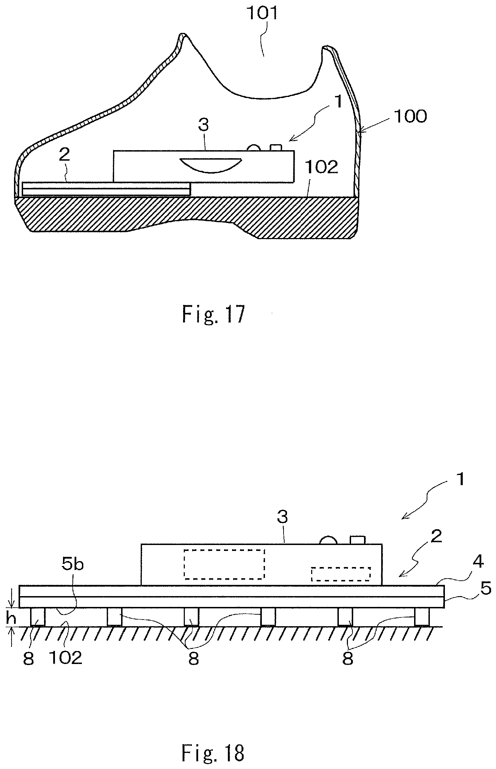

Fig. 17 is a schematic sectional view showing a usage example of the shoe cleaner.

The shoe cleaner 1 of the present embodiment is smaller in size than the shoe cleaner of the first embodiment, so that, as shown in Fig. 17, the shoe cleaner 1 can be easily inserted into a shoe 100.

Then, by positioning the ozone generating body 2 of the shoe cleaner 1 at a toe side of the shoe 100, the toe side with numerous bacteria and intense odors can be intensively sterilized and deodorized.

Other aspects of the configuration, operations, and effects are the same as those of the first embodiment described above, and descriptions thereof are omitted.

### (Third Embodiment)

Next, a third embodiment of the present invention is described.

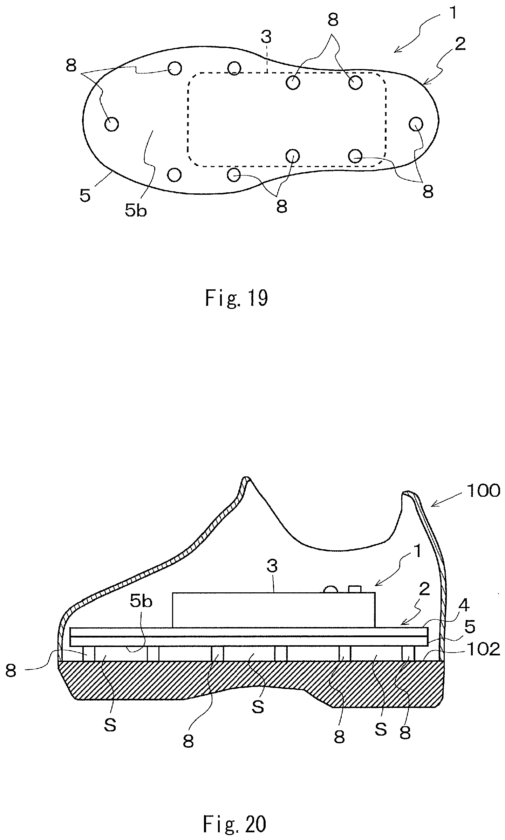

Fig. 18 is a side view of a shoe cleaner according to a third embodiment of the present invention, Fig. 19 is a plan view of the shoe cleaner, viewed from a back surface side, and Fig. 20 is a schematic sectional view showing a state where the shoe cleaner is inserted into a shoe.

As shown in Fig. 18, the shoe cleaner 1 of the present embodiment includes spacers 8 to keep a distance h between the back surface of the ozone generating body 2, that is, the back surface 5b of the electrode sheet 5 and the shoe sole 5b at a predetermined value or less.

That is, as shown in Fig. 19, a plurality of spacers 8 being projections are provided to project from the back surface 5b of the electrode sheet 5 of the ozone generating body 2. Accordingly, as shown in Fig. 20, when the shoe cleaner 1 is inserted into the shoe 100, a gap S is produced between the back surface 5b of the ozone generating body 2 and the shoe sole 102 by the spacers 8. As a result, a large amount of air is guided into the gap S, and ozone generation by the electrode sheet 5 becomes active.

Meanwhile, when the shoe cleaner 1 is inserted into the shoe 100 to sterilize and deodorize the shoe, in a case where the shoe sole 102 or an insole (not shown) slips, regardless of the distance between the ozone generating body 2 and the shoe sole 102, etc., bacteria on the shoe sole 102, etc., can be substantially entirely sterilized by ozone and deodorized.

However, in a case where the surfaces of the shoe sole 102 of the shoe 100 and the insole are rough, or the shoe sole 102 and the insole are formed of rough-textured materials such as cloth or artificial leather, when the distance between the surface of the shoe sole 102 or the insole and the electrode sheet 5 of the ozone generating body 2 is large, the sterilization effect may be hardly obtained.

Therefore, in the present embodiment, a height h of the spacers 8 is set within a range not more than 10 mm so that the rough shoe sole 102, etc., to be sterilized are positioned near the electrode sheet 5.

Accordingly, ozone and radicals, etc., generated from the back surface 5b of the electrode sheet 5 effectively come into contact with the shoe sole 102, etc., and high sterilization and deodorization effects are obtained.

The inventors conducted the following experiment to prove the above-described effects.

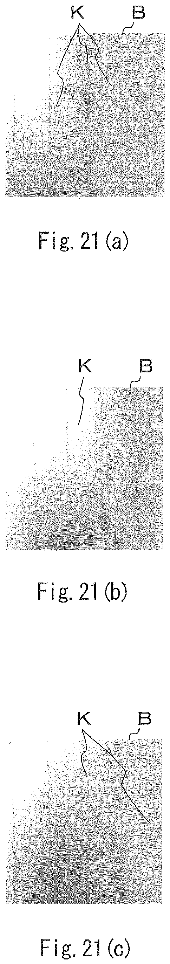

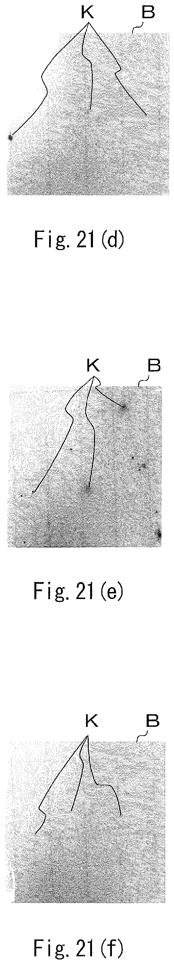

Figs. 21 are transferred images of photographs showing survival states of bacteria K.

The inventors conducted this experiment in a general indoor environment.

First, as preparation, bacteria (common bacteria, hereinafter, referred to as "bacteria K") were separately cultured, and the bacteria K were sprayed onto a polyester cotton blend cloth piece not shown in the drawings, and then the cloth piece was dried.

Then, the surface of the thus prepared cloth piece was wiped with a 5cm-square culture medium B, and the bacteria K were cultured in this culture medium B for 48 hours at a temperature of 35 °C. Then, as shown in Fig. 21(a), "137" bacteria K survived in the 5cm-square culture medium B.

That is, it was confirmed that, in the state where ozone treatment was not applied, the number of surviving bacteria K was "137."

Next, the prepared cloth piece was fixed at a position xmm just below the electrode sheet 5 in a state where the surface onto which the bacteria K were sprayed was made to face the electrode sheet 5. In this state, ozone was generated from the electrode sheet 5, and the cloth piece was exposed to ozone for 2 hours. At this time, the ozone was generated by supplying a pulsed power of 14 kV p-p at a frequency of 13 Hz to the electrode sheet 5.

Thereafter, the surface of the cloth piece to which ozone treatment was applied was wiped with a 5cm-square culture medium B, and the bacteria K were cultured in this culture medium B for 48 hours at a temperature of 35 °C.

The ozone treatment described above was applied by setting the distance xmm between the cloth piece and the electrode sheet 5 to 2 mm, 5mm, 10 mm, 20 mm, and 30 mm. Then, when the distance between the cloth piece and the electrode sheet 5 was 2 mm, as shown in Fig. 21(b), the number of surviving bacteria K in the 5cm-square culture B was "1," and when the distance between the cloth piece and the electrode sheet 5 was 5 mm, as shown in Fig. 21(c), the number of surviving bacteria K in the 5cm-square culture B was "19," and when the distance between the cloth piece and the electrode sheet 5 was 10 mm, as shown in Fig. 21(d), the number of surviving bacteria K in the 5cm-square culture B was "51," and when the distance between the cloth piece and the electrode sheet 5 was 20 mm, as shown in Fig. 21 (e), the number of surviving bacteria K in the 5cm-square culture B was "103," and when the distance between the cloth piece and the electrode sheet 5 was 30 mm, as shown in Fig. 21(f), the number of surviving bacteria K in the 5cm-square culture B was "102."

That is, it was confirmed that in the state where ozone treatment was applied, when the distance between the cloth piece and the electrode sheet 5 was increased, the number of surviving bacteria K increased.

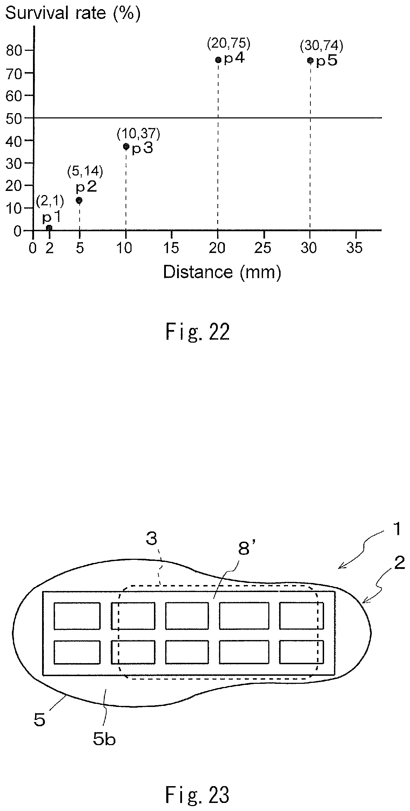

Fig. 22 is a diagram showing a relationship between a survival rate of the bacteria K and the distance.

The inventors defined the survival rate of the bacteria K as (the number of bacteria after ozone treatment/the number of bacteria without ozone treatment) × 100 (%), and plotted the relationship between the distance from the electrode sheet 5 to the cloth piece and the survival rate of the bacteria K in the ozone treatment described above.

Then, as shown in Fig. 22, points p1 to p5 were obtained.

As shown by the points p1, p2, and p3, when the distance was 2 mm, 5 mm, and 10 mm, the survival rate was 1%, 14%, and 37%, and these are lower than a reference survival rate of 50 %.

However, as shown by the points p4 and p5, when the distance was 20 mm and 30 mm, the survival rate was 75% and 74%, and these are much higher than the reference survival rate of 50%.

From this experimental result, it can be judged that a distance between the cloth piece and the electrode sheet 5 to reliably reduce the survival rate to be less than 50 % is 10 mm or less.

Therefore, the inventors set the distance h to be kept by the spacers 8 shown in Fig. 18 to be in a range of 10 mm or less. By thus keeping the distance h between the shoe sole 102, etc., and the electrode sheet 5 in the range of 10 mm or less, as shown in Fig. 22, the bacteria K surviving on the surface of the shoe sole 102, etc., can be reduced to be less than the reference survival rate of "50" %.

Other aspects of the configuration, operations, and effects are the same as those of the first or second embodiment described above, and descriptions thereof are omitted.

### (Fourth Embodiment)

Next, a fourth embodiment of the present invention is described.

Fig. 23 is a plan view of a shoe cleaner according to a fourth embodiment of the present invention, viewed from the back surface.

As shown in Fig. 23, the present embodiment is different from the third embodiment described above in that the spacer is formed into not a projection shape but a mesh-like body.

In detail, a spacer 8' is lattice-shaped, and attached to the back surface 5b of the electrode sheet 5. The spacer 8' is set to a thickness of 10 mm or less.

The spacer 8' is only required to be a mesh-like body. That is, not only the lattice-shaped plate body but also a plate body with multiple holes or a mesh body formed of thin wires can also be applied as the spacer 8'.

Other aspects of the configuration, operations, and effects are the same as those of the third embodiment described above, and descriptions thereof are omitted.

### (Fifth Embodiment)

Next, a fifth embodiment of the present invention is described.

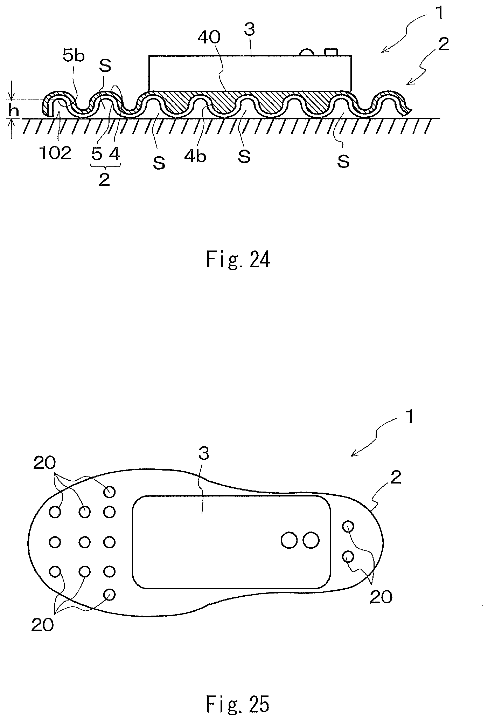

Fig. 24 is a side view showing a shoe cleaner according to a fifth embodiment of the present invention.

In Fig. 24, for easy viewing of the configuration of the shoe cleaner 1, the side surface of the substrate 4 of the ozone generating body 2 is hatched.

In the present embodiment, the substrate 4 was curved to become corrugated, and the electrode sheet 5 was stuck to the back surface 4b of the substrate 4 to follow the shape of the substrate 4. At this time, a height h of corrugation of the ozone generating body 2 (height to the back surface 5b of the electrode sheet 5) was set to 10 mm or less. Then, a flat portion 40 was formed in a central portion on the front surface side of the substrate 4, and the case body 3 is attached onto the flat portion 40.

With this configuration, when the shoe cleaner 1 is inserted into the shoe 100 (refer to Fig. 7, etc.), a large number of gaps S are formed between the shoe sole 102 of the shoe 100 and the back surface 5b of the corrugated electrode sheet 5, and these gaps S are filled with a large amount of air. Accordingly, the ozone generating efficiency of the electrode sheet 5 is improved.

Further, the height of corrugation of the ozone generating body 2 is set to 10 mm or less, so that the shoe sole 102 and an insole, etc., even when formed of rough-textured materials, cloth, or artificial leather, can also be effectively sterilized and deodorized.

In addition, the electrode sheet 5 is corrugated, so that the back surface of the electrode sheet 5 has an area larger than that of a planar electrode sheet.

Other aspects of the configuration, operations, and effects are the same as those of the first to fourth embodiments described above, and descriptions thereof are omitted.

### (Sixth Embodiment)

Next, a sixth embodiment of the present invention is described.

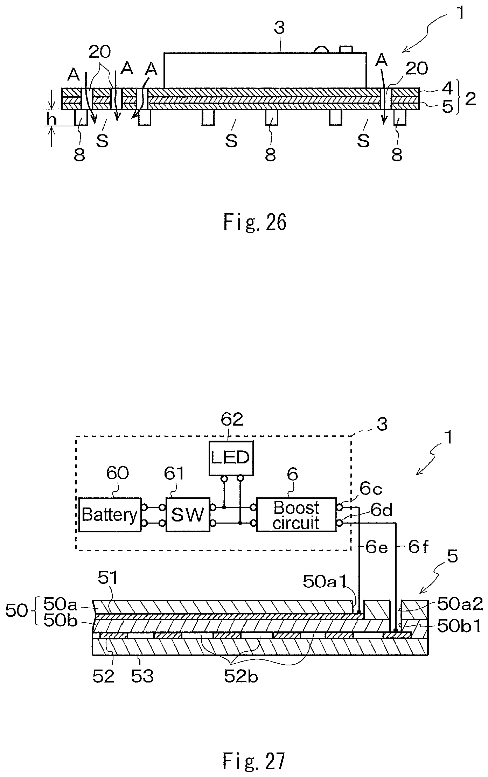

Fig. 25 is a plan view of a shoe cleaner according to a sixth embodiment of the present invention, and Fig. 26 is a side view showing the part of an ozone generating body in section.

As shown in Fig. 25 and Fig. 26, the shoe cleaner 1 of the present embodiment includes, as in the third embodiment, a plurality of spacers 8 with a height h of 10 mm or less on the back surface of the ozone generating body 2. Further, in the shoe cleaner 1 of the present embodiment, a plurality of circular holes 20 penetrating through the substrate 4 and the electrode sheet 5 from the front surface side to the back surface side are provided in the ozone generating body 2.

With this configuration, as shown in Fig. 26, a large amount of air A enters into the back surface side from the front surface side of the ozone generating body 2 through the plurality of holes 20. As a result, in the electrode sheet 5 of the ozone generating body 2, ozone is easily generated, so that the sterilization and deodorization effects can be improved.

In the present embodiment, as the holes, the circular holes 20 are applied, however, the holes are not limited to be circular, and polygonal holes and mesh-like holes may also be applied as the holes.

Other aspects of the configuration, operations, and effects are the same as those of the first to fifth embodiments described above, and descriptions thereof are omitted.

### (Seventh Embodiment)

Next, a seventh embodiment of the present invention is described.

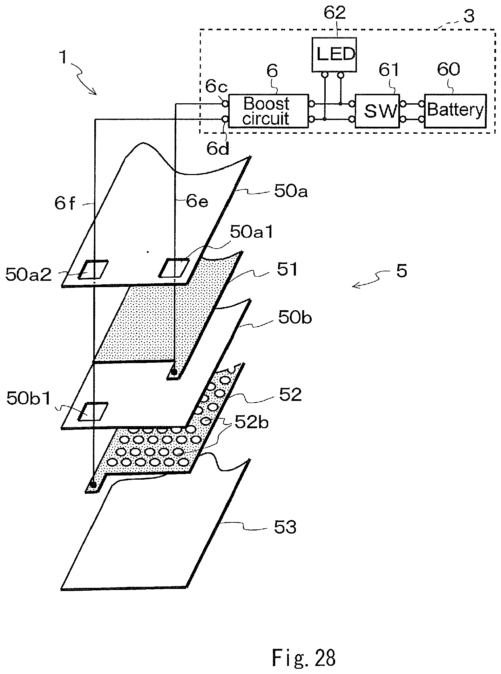

Fig. 27 is a schematic sectional view showing an essential portion of a shoe cleaner according to a seventh embodiment of the present invention, and Fig. 28 is an exploded perspective view of the essential portion shown in Fig. 27.

As shown in Fig. 27, in the shoe cleaner 1 of the present embodiment, the electrode sheet 5 has a structure different from that of the electrode sheets of the embodiments described above.

That is, only the electrode 51 as one electrode is housed inside the dielectric 50, and the electrode 52 as the other electrode is provided outside the dielectric 50.

In detail, as shown in Fig. 28, a solid-pattern electrode 51 is laminated on a dielectric layer 50b being a lower layer, and a dielectric layer 50a being an upper layer is laminated on the dielectric layer 50b so as to cover the electrode 51. An electrode 52 having substantially the same shape as the electrode 51 is formed under the dielectric layer 50b. Further, a protective layer 53 is formed under the electrode 52.

Moreover, in the dielectric layer 50a, a power supply port 50a1 exposing the electrode 51 is formed, and a wiring 6e extending from the output terminal 6c of the boost circuit 6 passes through this power supply port 50a1 and is connected to the electrode 51.

On the other hand, in the dielectric layer 50b, a power supply port 50b1 exposing the electrode 52 is formed, and at a portion of the dielectric layer 50a just above the power supply port 50b1, a power supply port 50a2 communicating with the power supply port 50b1 is formed. A wiring 6f extending from the output terminal 6d of the boost circuit 6 passes through the power supply ports 50a1 and 50b1 and is connected to the electrode 52.

In this electrode 52, a large number of circular holes 52b are opened at fixed intervals.

In the present embodiment, the protective layer 53 is provided under the electrode 52 located on a lower surface of the dielectric 50, however, the protective layer 53 is not an essential member, and does not need to be provided according to circumstances.

Other aspects of the configuration, operations, and effects are the same as those of the first to sixth embodiments described above, and descriptions thereof are omitted.

### (Sixth Modification)

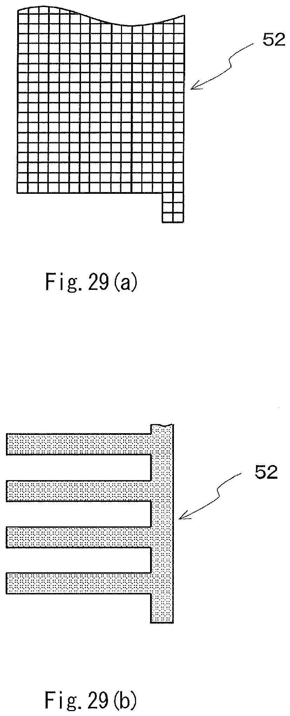

Figs. 29 are plan views showing modifications of the electrode applied in the seventh embodiment, in which Fig. 29(a) shows a first modification of the seventh embodiment, and Fig. 29(b) shows a second modification of the seventh embodiment.

In the seventh embodiment described above, as shown in Fig. 28, the electrode 52 having a large number of circular holes 52b is applied as the other electrode. However, as shown in Fig. 29(a), a meshed electrode 52 can be applied as the other electrode. As shown in Fig. 29(b), a comb-shaped electrode 52 may also be applied as the other electrode.

The embodiments described above each show an example in which the substrate 4 and the electrode sheet 5 forming the ozone generating body 2 are formed of flexible materials. However, a shoe cleaner including an ozone generating body 2 in which either or both of the substrate 4 and the electrode sheet 5 are formed of an inflexible material is also included in the scope of the present invention.

The third to fifth embodiments described above each show an example in which the height or thickness of the spacer 8, 8' or the corrugation height of the ozone generating body 2 is set to 10 mm or less. However, a shoe cleaner in which the height or thicknesses of the spacer 8, 8' or the corrugation height of the ozone generating body 2 is more than 10 mm is also included in the scope of the present invention.

The fifth embodiment described above shows an example in which a flat portion 40 is provided on the substrate 4, and the case body 3 is attached to this flat portion 40. However, as a matter of course, a structure may also be adopted in which the flat portion 40 is not provided on the substrate 4, and the case body 3 is directly attached onto the corrugated substrate 4.

Further, in the electrode sheet 5 of the first to sixth embodiments described above, a configuration in which a pair of comb-shaped electrodes 51 and 52 are housed together inside the dielectric 50 is adopted, and in the electrode sheet 5 of the seventh embodiment, a configuration in which one electrode 51 is housed inside the dielectric 50 and the other electrode 51 is provided on a lower surface of the dielectric 50, is applied. However, the configuration of the electrode sheet 5 is not limited to these. A configuration in which, for example, a pair of electrodes are formed into tabular shapes, and these two electrodes are arranged side by side at a fixed interval kept between them, may also be adopted, or a configuration in which the respective electrodes are formed into spiral shapes, and the pair of spiral-shaped electrodes are fitted to each other while keeping a fixed interval between these, may also be adopted.

### Reference Signs List

1 ... shoe cleaner, 2, 2-1, 2-2 ... ozone generating body, 2A ... toe side portion, 2B ... heel side portion, 2C ... arch side portion, 3 ... case body, 4 ... substrate, 4a, 5a ... front surface, 4b, 5b ... back surface, 5 ... electrode sheet, 6 ... boost circuit, 6a, 6b ... input terminal, 6c, 6d ... output terminal, 6e, 6f ... wiring, 7 ... ring, 8, 8' ... spacer, 20 ... hole, 30 ... bottom wall, 31 ... ceiling wall, 32, 33 ... side wall, 32a, 33a ... recess portion, 34 ... rear wall portion, 40 ... flat portion, 41, 42 ... joint member, 50 ... dielectric, 50a, 50b ... dielectric layer, 50a1, 50b1, 50a2 ... power supply port, 51, 52 ... electrode, 51a, 52a ... comb, 52b ... circular hole, 53 ... protective layer, 60 ... battery, 61 ... switch, 61a ... push button, 62 ... LED lamp, 63 ... timer, 100 ... shoe, 101 ... wearing opening, 102 ... shoe sole, 103 ... shoe lining, 110 ... floor, A ... air, B ... culture medium, h ... height (distance), K ... bacteria, M ... central axis, O₃ ... ozone, S ... gap

## Claims

1. A shoe cleaner (1) comprising: an ozone generating body (2) capable of being inserted into a shoe from a wearing opening of the shoe; and a case body (3) capable of being inserted into the shoe integrally with the ozone generating body (2), the shoe cleaner being **characterized in that**:
the case body (3) is attached to a front surface of the ozone generating body (2),
the ozone generating body (2) is formed of a sheet-like substrate (4) having the front surface (4a) attached to a bottom portion (30) of the case body (3), and an electrode sheet (5) having a front surface (5a) stuck to a back surface (4b) of the substrate (4), and including a pair of electrodes (51, 52), at least one of the pair of electrodes (51, 52) being covered by a dielectric (50) and at least the dielectric (50) being formed of a polymeric resin, and
a voltage supply portion (6) capable of supplying a voltage necessary to generate ozone to the pair of electrodes (51, 52) of the electrode sheet (5) is housed inside the case body (3).

2. The shoe cleaner (1) according to claim 1, wherein
at least one of the electrode sheet (5) and the substrate (4) is formed of a flexible material.

3. The shoe cleaner according to claim 1 or 2, wherein
the dielectric of the electrode sheet is formed of a polymeric resin with a relative permittivity of 3 or more and a dielectric breakdown voltage of 15 kV/mm or more.

4. The shoe cleaner (1) according to any of claims 1 to 3, wherein
the substrate (4) is formed of a material with a Young's modulus of 40 MPa or less.

5. The shoe cleaner (1) according to any of claims 1 to 4, wherein
a spacer (8) to keep a distance of 10 mm or less between a back surface (5b) of the ozone generating body (2) and a shoe sole is provided on the back surface (5b) of the ozone generating body (2).

6. The shoe cleaner (1) according to claim 5, wherein
the spacer (8) is a projection with a height of 10 mm or less provided to project from the back surface (5b) of the electrode sheet (5).

7. The shoe cleaner (1) according to claim 5, wherein
the spacer (8) is a mesh-like body with a thickness of 10 mm or less attached to the back surface (5b) of the electrode sheet (5).

8. The shoe cleaner (1) according to any of claims 1 to 4, wherein
a sectional shape of the ozone generating body (2) is corrugated.

9. The shoe cleaner (1) according to claim 8, wherein
a height of corrugation of the ozone generating body (2) is set to 10 mm or less.

10. The shoe cleaner (1) according to any of claims 1 to 9, wherein
the ozone generating body (2) is provided with a plurality of holes (20) penetrating through the substrate (4) and the electrode sheet (5) from the front surface side to the back surface side.

11. The shoe cleaner (1) according to any of claims 1 to 10, wherein
the pair of electrodes (51, 52) in the electrode sheet (5) are respectively formed into comb shapes, and combs of the pair of electrodes (51a, 52a) engage with each other while keeping a fixed interval between them.

12. The shoe cleaner (1) according to any of claims 1 to 10, wherein one of the pair of electrodes (51) in the electrode sheet (5) is housed inside the dielectric (50a, 50b), and either the other electrode having a large number of holes or the other electrode having a comb shape (52) is located on the dielectric so as to face the one electrode.

## Patentansprüche

1. Schuhreiniger (1), welcher aufweist: einen Ozon erzeugenden Körper (2), der in einen Schuh eingeführt werden kann über eine Trageöffnung des Schuhs; und einen Gehäusekörper (3), der in den Schuh eingeführt werden kann einstückig mit dem Ozon erzeugenden Körper (2), wobei der Schuhreiniger **dadurch gekennzeichnet ist, dass**:
der Gehäusekörper (3) an einer vorderen Oberfläche des Ozon erzeugenden Körpers (2) angeordnet ist,
der Ozon erzeugende Körper (2) gebildet ist aus einem blattförmigen Substrat (4), wobei die vordere Oberfläche (4a) an einem unteren Abschnitt (30) des Gehäusekörpers (3) angeordnet ist, und einem Elektrodenblatt (5), wobei die vordere Oberfläche (5a) an eine hintere Oberfläche (4b) des Substrats (4) geklebt ist, und ein Elektrodenpaar (51, 52) aufweist, mindestens eine Elektrode des Elektrodenpaars (51, 52) von einem Dielektrikum (50) bedeckt wird und mindestens das Dielektrikum (50) aus einem Polymerharz gebildet wird, und
eine Spannungsversorgungseinrichtung (6), die in der Lage ist, dem Elektrodenpaar (51, 52) des Elektrodenblatts (5) eine zur Erzeugung von Ozon erforderliche Spannung zuzuführen, im Inneren des Gehäusekörpers (3) untergebracht ist.

2. Schuhreiniger (1) nach Anspruch 1, wobei
mindestens eines von dem Elektrodenblatt (5) und dem Substrat (4) aus einem flexiblen Material gebildet ist.

3. Schuhreiniger nach Anspruch 1 oder 2, wobei
das Dielektrikum des Elektrodenblatts gebildet ist aus einem Polymerharz mit einer relativen Permittivität von 3 oder mehr und einer dielektrischen Durchbruchspannung von 15 kV/mm oder mehr.

4. Schuhreiniger (1) nach irgendeinem der Ansprüche 1 bis 3, wobei das Substrat (4) gebildet ist aus einem Material mit einem Elastizitätsmodul von 40 MPa oder weniger.

5. Schuhreiniger (1) nach irgendeinem der Ansprüche 1 bis 4, wobei
ein Abstandshalter (8) zum Einhalten eines Abstands von 10 mm oder weniger zwischen einer hinteren Oberfläche (5b) des Ozon erzeugenden Körpers (2) und einer Schuhsohle auf der hinteren Oberfläche (5b) des Ozon erzeugenden Körpers (2) vorgesehen ist.

6. Schuhreiniger (1) nach Anspruch 5, wobei
der Abstandshalter (8) ein Vorsprung mit einer Höhe von 10 mm oder weniger ist, der vorgesehen ist, um von der hinteren Oberfläche (5b) des Elektrodenblatts (5) vorzustehen.

7. Schuhreiniger (1) nach Anspruch 5, wobei
der Abstandshalter (8) ein netzartiger Körper mit einer Dicke von 10 mm oder weniger ist, der an der hinteren Oberfläche (5b) des Elektrodenblatts (5) angeordnet ist.

8. Schuhreiniger (1) nach irgendeinem der Ansprüche 1 bis 4, wobei
eine Schnittform des Ozon erzeugenden Körpers (2) gewellt ist.

9. Schuhreiniger (1) nach Anspruch 8, wobei
eine Wellenhöhe des Ozon erzeugenden Körpers (2) auf 10 mm oder weniger eingestellt ist.

10. Schuhreiniger (1) nach irgendeinem der Ansprüche 1 bis 9, wobei
der Ozon erzeugende Körper (2) versehen ist mit einer Mehrzahl von Löchern (20), die das Substrat (4) und das Elektrodenblatt (5) von der vorderen Oberflächenseite zu der hinteren Oberflächenseite durchdringen.

11. Schuhreiniger (1) nach irgendeinem der Ansprüche 1 bis 10, wobei
das Elektrodenpaar (51, 52) in dem Elektrodenblatt (5) jeweils zu Kamm-Formen ausgebildet sind und Kämme des Elektrodenpaares (51a, 52a) ineinander eingreifen, wobei ein fester Abstand zwischen ihnen eingehalten wird.

12. Schuhreiniger (1) nach irgendeinem der Ansprüche 1 bis 10, wobei
eine Elektrode des Elektrodenpaares (51) in dem Elektrodenblatt (5) im Inneren des Dielektrikums (50a, 50b) untergebracht ist und entweder die andere Elektrode mit einer großen Anzahl von Löchern oder die andere Elektrode, welche eine Kamm-Form (52) aufweist, auf dem Dielektrikum so angeordnet ist, dass sie der einen Elektrode zugewandt ist.

## Revendications

1. Dispositif de nettoyage de chaussure (1) comprenant : un corps de génération d'ozone (2) capable d'être inséré dans une chaussure à partir d'une ouverture d'utilisation de la chaussure ; et un corps d'enveloppe (3) capable d'être inséré dans la chaussure d'un seul tenant avec le corps de génération d'ozone (2), le dispositif de nettoyage de chaussure étant **caractérisé en ce que** :
le corps d'enveloppe (3) est fixé à une surface avant du corps de génération d'ozone (2),
le corps de génération d'ozone (2) est formé d'un substrat analogue à une feuille (4) présentant la surface avant (4a) fixée à une partie inférieure (30) du corps d'enveloppe (3), et d'une feuille d'électrode (5) présentant une surface avant (5a) collée à une surface arrière (4b) du substrat (4), et incluant une paire d'électrodes (51, 52), au moins une de la paire d'électrodes (51, 52) étant recouverte par un diélectrique (50) et au moins le diélectrique (50) étant formé d'une résine polymère, et
une partie d'alimentation en tension (6) capable de fournir une tension nécessaire pour générer de l'ozone à la paire d'électrodes (51, 52) de la feuille d'électrode (5) est logée à l'intérieur du corps d'enveloppe (3).

2. Dispositif de nettoyage de chaussure (1) selon la revendication 1, dans lequel
au moins l'un de la feuille d'électrode (5) et du substrat (4) est formé d'un matériau flexible.

3. Dispositif de nettoyage de chaussure selon la revendication 1 ou 2, dans lequel
le diélectrique de la feuille d'électrode est formé d'une résine polymère avec une permittivité relative de 3 ou plus et une tension de claquage diélectrique de 15 kV/mm ou plus.

4. Dispositif de nettoyage de chaussure (1) selon l'une quelconque des revendications 1 à 3, dans lequel
le substrat (4) est formé d'un matériau avec un module de Young de 40 MPa ou moins.

5. Dispositif de nettoyage de chaussure (1) selon l'une quelconque des revendications 1 à 4, dans lequel
un élément d'espacement (8) pour maintenir une distance de 10 mm ou moins entre une surface arrière (5b) du corps de génération d'ozone (2) et une semelle de chaussure est prévue sur la surface arrière (5b) du corps de génération d'ozone (2).

6. Dispositif de nettoyage de chaussure (1) selon la revendication 5, dans lequel
l'élément d'espacement (8) est une saillie d'une hauteur de 10 mm ou moins prévue pour faire saillie à partir de la surface arrière (5b) de la feuille d'électrode (5).

7. Dispositif de nettoyage de chaussure (1) selon la revendication 5, dans lequel
l'élément d'espacement (8) est un corps analogue à un treillis d'une épaisseur de 10 mm ou moins fixé à la surface arrière (5b) de la feuille d'électrode (5).

8. Dispositif de nettoyage de chaussure (1) selon l'une quelconque des revendications 1 à 4, dans lequel
une forme en coupe du corps de génération d'ozone (2) est ondulée.

9. Dispositif de nettoyage de chaussure (1) selon la revendication 8, dans lequel
une hauteur d'ondulation du corps de génération d'ozone (2) est réglée à 10 mm ou moins.

10. Dispositif de nettoyage de chaussure (1) selon l'une quelconque des revendications 1 à 9, dans lequel
le corps de génération d'ozone (2) est pourvu d'une pluralité de trous (20) pénétrant à travers le substrat (4) et la feuille d'électrode (5) depuis le côté de surface avant vers le côté de surface arrière.

11. Dispositif de nettoyage de chaussure (1) selon l'une quelconque des revendications 1 à 10, dans lequel
la paire d'électrodes (51, 52) dans la feuille d'électrode (5) sont respectivement formées en formes de peigne, et les peignes de la paire d'électrodes (51a, 52a) viennent en prise mutuellement tout en conservant un intervalle fixe entre eux.

12. Dispositif de nettoyage de chaussure (1) selon l'une quelconque des revendications 1 à 10, dans lequel
une première de la paire d'électrodes (51) dans la feuille d'électrode (5) est logée à l'intérieur du diélectrique (50a, 50b), et l'autre électrode présentant un grand nombre de trous ou l'autre électrode présentant une forme de peigne (52) est située sur le diélectrique de manière à faire face à la première électrode.
